# EUROPEAN PATENT SPECIFICATION

(11) **EP 1 603 807 B1**
(45) Date of publication and mention of the grant of the patent: **12.07.2006**
(21) Application number: 03779863.4
(22) Date of filing: 05.11.2003
(51) Int. Cl.: B65D 43/16, B65D 83/14

(54) **CONTAINER WITH A HINGE**
BEHÄLTER MIT SCHARNIER
RECIPIENT A CHARNIÈRE

(30) Priority: 07.11.2002 GB 0226021
(43) Date of publication of application: 14.12.2005
(73) Proprietor: GLAXO GROUP LIMITED, Greenford, Middlesex UB6 ONN (GB)
(72) Inventor: DAVIES, Michael Birsha, Hertfordshire SG12 0DP (GB); GODFREY, James William, Ware, Hertfordshire SG12 0DP (GB); HARVEY, Stephen James, Ware, Hertfordshire SG12 0DP (GB); RAND, Paul Kenneth, Ware, Hertfordshire SG12 0DP (GB)
(74) Representative: Rice, Jason Neale
(86) International application number: PCT/EP2003/012435
(87) International publication number: WO 2004/041670

(56) References cited:
- US-A- 3 865 279

## Description

### Field of the Invention

The present invention relates to a container, and is particularly, but not exclusively, concerned with a container for use in a medicament dispensing system.

### Background of the Invention

There is previously known an aerosol nasal inhalation (intranasal) device in which an aerosol canister containing a drug formulated in a pressurised fluid propellant is mounted in an outer protective container. Such a nasal inhaler is marketed under the trade name Beconase® (GlaxoSmithKilne) with the drug being beclomethasone dipropionate.

The canister has a canister body holding the drug formulation which is capped with a valve assembly comprising a valve stem through which the drug formulation is selectively discharged from the canister body. The valve stem is biased to an extended (closed) position relative to the canister body by a spring or the like in the valve assembly. In the extended position the valve stem places the drug formulation in fluid communication with a metering chamber in the valve assembly. This is the metering state and the metering chamber is seated-from the external environment in this state. The valve stem is movable against the biasing force to a retracted (open) position relative to the canister body. In the retracted position the valve stem places the metering chamber in fluid communication with the external environment whereby the metered quantity of the drug formulation in the metering chamber is exhausted from the canister for administration to the patient. This is the discharge state and in this state the metering chamber is sealed from the interior of the canister body.

The container has a tube-like container part with a cavity which receives the canister therein. As known in the art, a base of the cavity presents a hollow stem block to receive the valve stem in stationary relation thereto. A nozzle sized to be received in a patient's nostril extends outwardly from the outer surface of the container part. The stem block has an orifice which is oriented towards the nozzle.

In use, the patient depresses the canister into the cavity of the container part thereby resulting in the canister body moving relative to the valve stem to bring the valve stem to its retracted position. This causes a metered amount of the drug formulation to be discharged from the valve stem and directed out of the nozzle by the stem block to be delivered in the patient's nostril.

In addition to the container part, the container has a closure part which is hinged to the mouth area of the cavity of the container part so as to be hingeable from a closed position, in which the closure part co-operates with the container part to enclose the canister and the nozzle, to an open position in which the closure and container parts are arranged "back-to-back" to expose the canister and nozzle. In the closed position the container acts as a protective casing for the inhalation device, while in the open position the patient can use the container as an applicator or holder for gripping the inhalation device when administering the drug.

A prior art container in accordance with the preamble of appended claim 1 is disclosed in US 3 865 279.

It is an aim of the present invention to provide a new multi-part container, e.g. for a medicament dispensing system.

### Summary of the Invention

According to the present invention there is provided a container having a first part, a second part and a hinge through which the first and second parts are hingeably connected so that the parts are hingeable relative to one another between a first position which places the container in a closed state and a second position which places the container in an open state, characterised in that the first and second parts are further pivotally connected so that the parts are able to be pivoted relative to one another to different angular positions. The first and second parts may be container and lid parts, respectively, perhaps of shell-like form, such as in the embodiment hereinafter to be described. Preferably, the parts are formed from a plastics material, preferably by moulding, for example injection moulding.

In an embodiment of the invention, such as hereinafter described, the container is adapted such that the first and second parts are not pivotal relative to one another when the container is in the closed state. To this end, in the closed state the first and second parts may interengage one another so as to prevent pivotal movement therebetween. The interengagement may be between respective lip structures of the first and second parts.

Preferably, the hinge is a living hinge, especially when the container is formed from a plastics material, e.g. by moulding, although other types of hinge may be used.

In an embodiment of the invention, such as hereinafter described, the first and second parts have locking elements which interengage in the closed state of the container to lock the container in the closed state. The locking elements may be such as to form a snap fit connection of the first and second parts in the closed state of the container.

In an embodiment of the invention, such as hereinafter described, the hinge is pivotally mounted to the second part for enabling the relative pivotal movement of the first and second parts.

In an embodiment of the invention, such as hereinafter described, the hinge is statically mounted to the first part.

In an embodiment of the invention, such as hereinafter described, the hinge forms an integral part of the first part and is pivotally mounted to the second part. In other words, the first part is pivotally mounted to the second part through the hinge. The first part may be releasably pivotally mounted to the second part, as, for example, in the embodiment hereinafter described.

In an embodiment of the invention, such as hereinafter described, the first part is pivotal to a first angular position disposed behind the second part. The first part may only be pivotal to the first angular position after the container is in the open state.

Preferably, the first and second parts are adapted to nest together in a nesting state when the first part is in the first angular position. The first part may be of hollow form into which the second part is receivable, at least in part, to form the nesting state. The second part may be hollow too, whereby in the closed state the respective hollows co-operate to form a product-receiving chamber enclosed by the first and second parts.

In an embodiment of the invention, such as hereinafter described, the second part has a rear surface in which is provided a reading feature, for example a window, the first part covers the reading feature in the first angular position and has a viewing feature to enable the reading feature to be viewed through the first part when in the first angular position. As an example, the first part is at least in part made from a material sufficiently transparent to enable the reading feature to be viewed therethrough when the first part is in the first angular position.

The container may further have a detent mechanism for detenting the first and second parts in different angular positions, for example an indexing mechanism based on interengagable male and female parts, such as a ball-and-socket indexing mechanism. In this way, the detent mechanism may be adapted to detent the first part in an angular position behind the second part which provides an unimpeded view of the reading feature on the second part.

Preferably, the first and second parts form an enclosing structure when in the closed state. The enclosing structure may be an outer structure with the container including an inner part releasably, fixably securable in one of the first and second parts so as to be enclosed by the outer structure.

In an embodiment of the invention, such as hereinafter described, the inner part is adapted to be snap-fitted in one of the first and second parts, conveniently in the second part.

The inner part may be adapted to hold a product in the container with the product also enclosed by the outer structure. The product may be a package with contents to be dispensed. In this case, the inner part may be a dispenser with a dispensing mechanism for dispensing the contents of the package.

In an embodiment of the invention, such as hereinafter described, the dispensing mechanism operates through relative movement between the dispenser and the package when the dispenser is fixedly secured in the outer structure.

In an embodiment of the invention, such as hereinafter described, the dispenser has a delivery nozzle through which the contents of the package are dischargeable.

In an embodiment of the invention, such as hereinafter described, the dispenser is an intranasal dispenser.

The package and dispenser may form a dispenser assembly with the package containing multiple doses of the contents and the dispenser assembly having a dose counter for counting the number of doses dispensed from the package, the dose counter being aligned with the reading feature when the dispenser assembly is secured in the container. The dose counter may be united with the package, as in the embodiment hereinafter described.

The package may be an aerosol canister with fluidic contents and having a valve openable by the dispensing mechanism on relative movement of the canister to the dispenser for release of a dose of the fluidic contents from the dispenser. As an example, the valve may have a stem through which the fluidic contents is dispensed from the dispenser on relative movement thereof to the canister and the dispenser has a stand adapted to receive the stem in static relation thereto whereby the canister is able to be moved relative to the stem (and the dispenser) when the stem is received in the stand for dispensing of the fluidic contents.

The contents of the package may be a pharmaceutical composition, for example a pharmaceutical composition for the treatment or prophylaxis of a respiratory disease or disorder, for instance rhinitis.

According to the present invention there is also provided a medicament dispensing system having the container of the invention.

Non-limiting exemplary embodiments of the invention will now be described with reference to the accompanying FIGURES of drawings.

### Brief Description of the Drawings of the Exemplary Embodiments

FIGURE 1 is a schematic view of a patient using an intranasal device having an outer casing part in accordance with the invention.
FIGURE 2 is a side view of the intranasal device showing the outer casing part which comprises cover and container members and a hinge therebetween, the outer casing part in a closed state to protect inner parts of the device.
FIGURE 3 is a side view of the intranasal device with the outer casing part hinged to an open state to allow access to the inner parts.
FIGURE 4A is a side view of the intranasal device in its open state with the cover member pivoted behind the container member
FIGURE 4B corresponds to FIGURE 4A, but with the container and cover members nested.
FIGURE 5 is a rear view of the device with the outer casing part in its closed state.
FIGURE 6 is an exploded perspective view of the intranasal device with the outer casing part in its nested state.
FIGURE 7 is a schematic, partial sectional side view of an inner actuating part of the intranasal device mounted in the outer casing part.
FIGURE 8 is a enlarged fragmentary view of an alternative hinge construction for the outer casing part.
FIGURE 9 is a cross-sectional side view of the alternative hinge construction along line IX-IX in FIGURE 8.
FIGURE 10A is a perspective view of a canister unit of the intranasal device comprising a canister and a dose counter head mounted on the canister.
FIGURE 10B is a plan view of the dose counter head.
FIGURE 10C is a side view of the dose counter head.
FIGURE 10D is a rear view of the dose counter head.
FIGURE 11 is schematic side view, partly,in section, of the inner actuating part of the intranasal device.
FIGURE 12 is an opposite side view of the inner actuating part with the canister unit shown mounted therein in ghost.
FIGURE 13 is a plan view of the inner actuating part.
FIGURE 14 is a rear view of the inner actuating part with the canister unit mounted therein.
FIGURE 15A is a schematic side view, partly in cross section, of the canister unit mounted in the inner actuating part in an inoperative position.
FIGURE 15B corresponds to FIGURE 15A, but with the canister unit in an operative position relative to the inner actuating part.

### Detailed Description of the Exemplary Embodiment

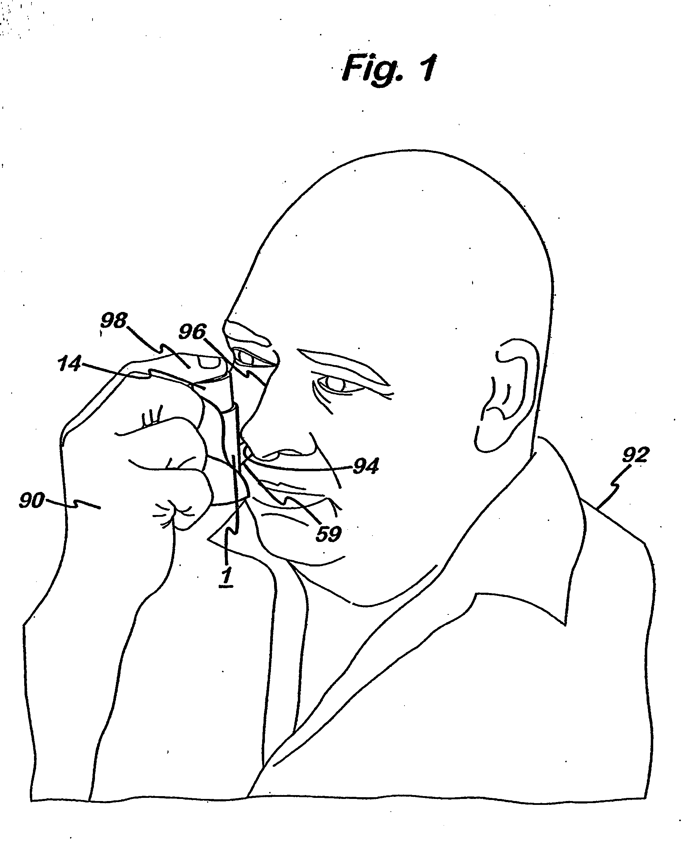

In the FIGURES of drawings there is shown a drug delivery device 1 in accordance with the present invention, the device 1 in this particular non-limiting embodiment being an intranasal drug delivery device, as shown graphically in FIGURE 1.

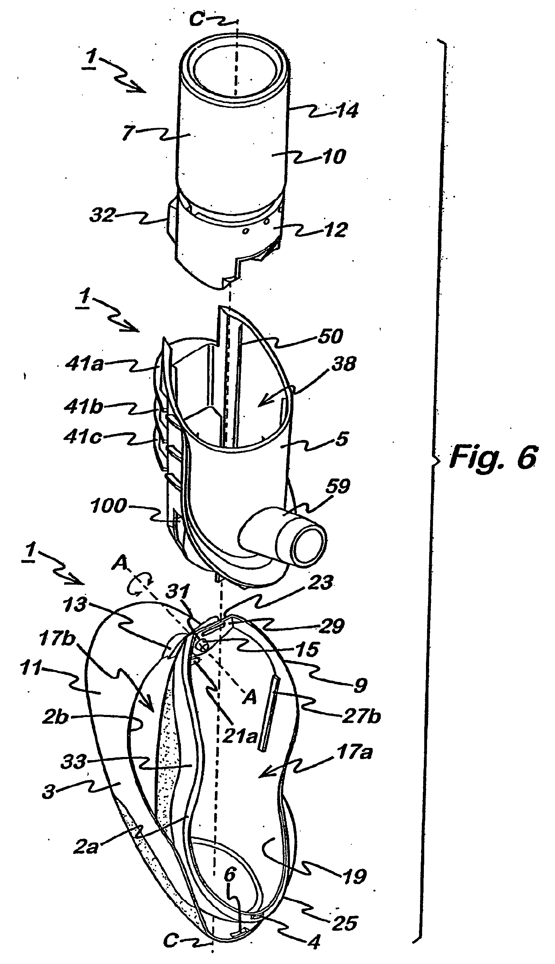

As best shown in FIGURE 6, the intranasal drug delivery device 1 comprises as component parts the following:-
- an outer casing part 3;
- a one-piece inner actuating part 5 which is releasably securable in the outer casing part 3; and
- a canister unit 7 releasably securable in the inner actuating part 5.

### Outer Casing Part

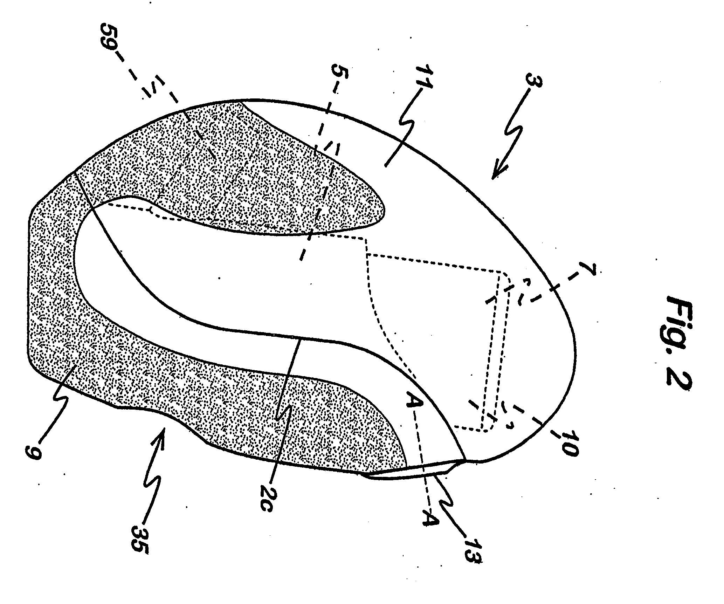

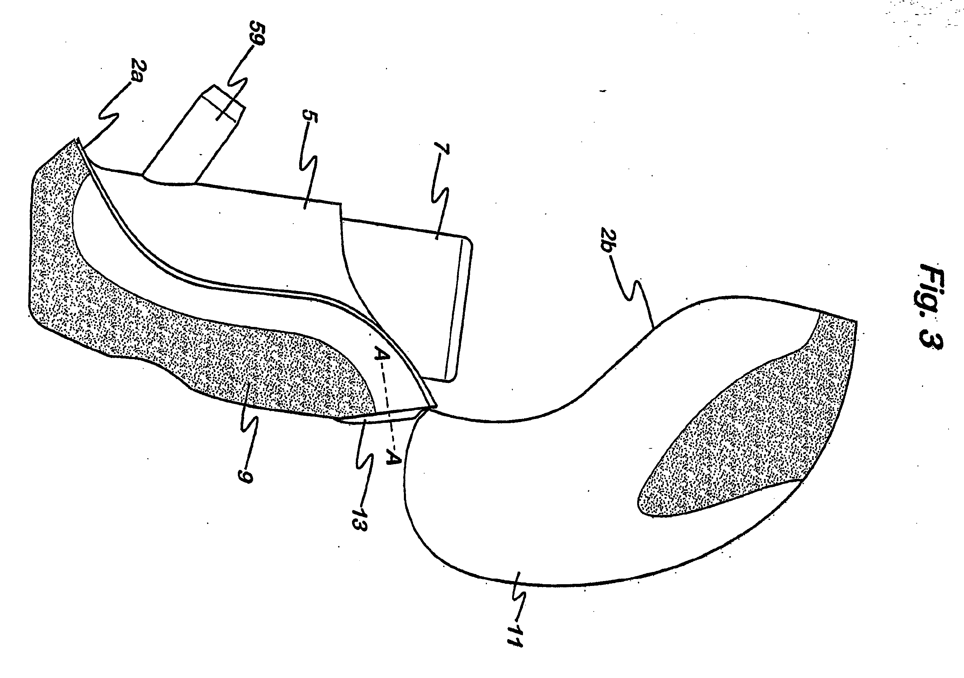

Referring to FIGURES 2 to 7, the outer casing part 3 of the intranasal device 1 is formed from two shell-like members, namely a container member 9 and a cover member 11, which are connected to one another through a hinge 13. The container and cover members 9, 11 each respectively present a cavity 17a; 17b having a mouth bound by a lip surface 2a; 2b (FIGURE 6).

Both the container member 9 and the cover member 11 are formed from a plastics material by injection moulding, although other types of moulding processes can, of course, be used. The container and cover members 9, 11 are preferably both formed from polypropylene with the container member 11 being opaque, but with the cover member 11 being transparent or semi-transparent. Other plastics material combinations are possible, although it is preferable for the cover member 11 to be transparent/semi-transparent for reasons which will become apparent hereinafter.

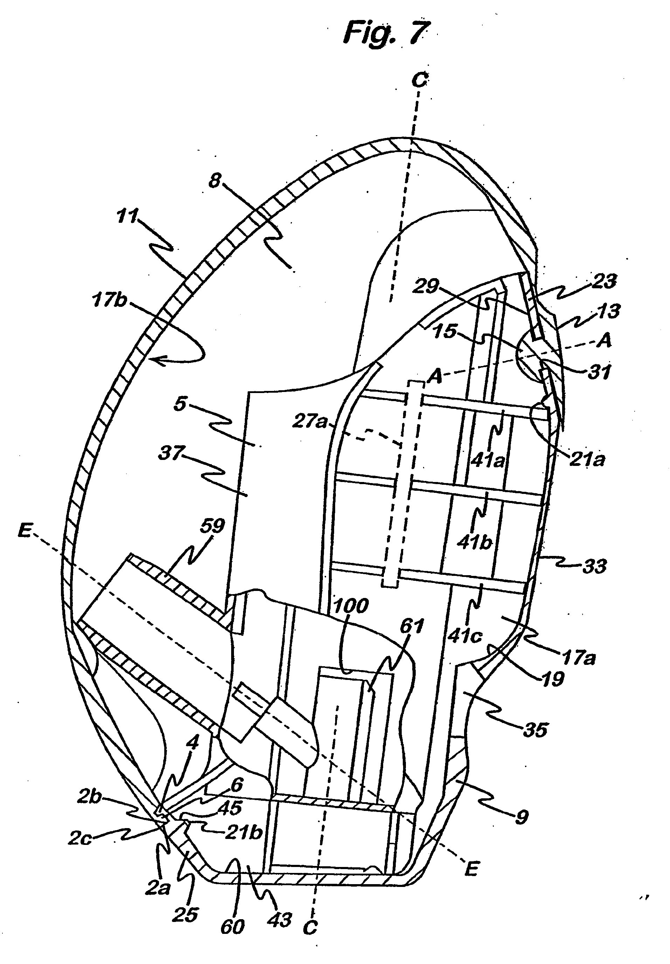

As shown in FIGURE 7, for example, the cover member 11 is integrally formed with the hinge 13, colloquially known as a "living hinge", and the hinge 13 includes a knob 15.

Turning to FIGURES 6 and 7, on an inner surface 19 of the cavity 17a of the shell-like container member 9 there is formed a pair of protrusions or ribs 21a, 21b to co-operate with complementary surfaces of the inner actuating part 5 to form a snap-fit connection between the outer casing part 3 and the inner actuating part 5, as will also be discussed in more detail hereinafter. A first one of the ribs 21a is disposed towards an upper end 23 of the container member 9, whereas the other rib 21b is disposed towards a lower end 25 of the container member 9.

Also formed on the inner surface 19 of the cavity 17a of the container member 9 are longitudinally extending ribs 27a, 27b. The longitudinal ribs 27a, 27b are disposed on opposing sides of the container member 9 and act as anti-rotational retainers on the inner actuating part 5 in the cavity 17a.

At the upper end 23 of the container member 9 there is formed a bevel 29 through which an aperture 31 extends from an outer surface 33 of the container member 9 to the inner surface 19 of the cavity 17a. From FIGURE 7 it will be seen that the aperture 31 is adapted to receive the knob 15 on the hinge 13. Although the diameter of the knob 15 is greater than the diameter of the aperture 31, the knob 15 is able to be pushed through the aperture 31 for capture therein due to the container and cover members 9, 11 being sufficiently resiliently deformable due to their shell-like nature and the materials used. Moreover, the knob 15 is able to be withdrawn from the aperture 31 upon application of a sufficient pulling force thereto for separation of the container and cover members 9, 11.

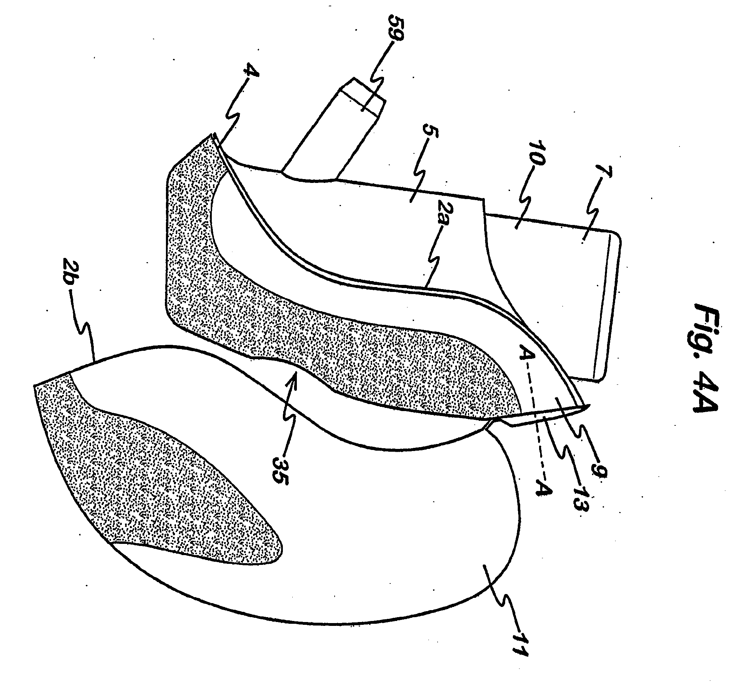

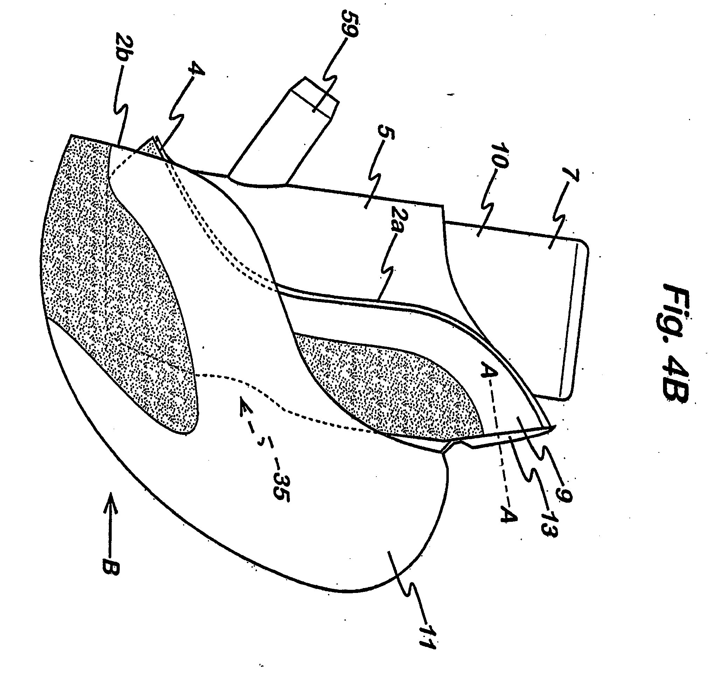

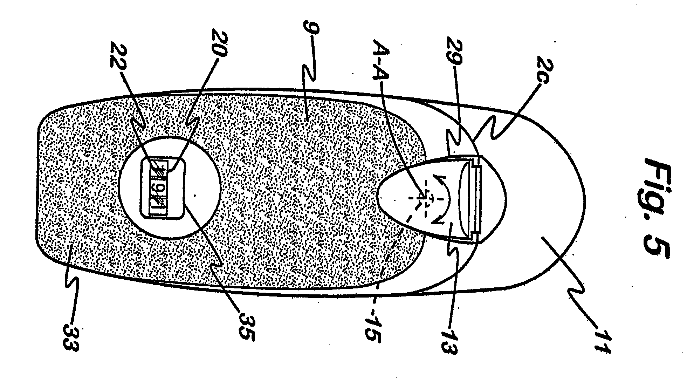

As shown in FIGURES 2 to 5, the mounting of the knob 15 of the hinge 13 in the aperture 31 of the container member 9 enables two degrees of movement of the cover member 11 on the container member 9 as follows:-
(1) Hinging of the cover member 11 from a closed position shown in FIGURES 2 and 5, in which the respective lip surfaces 2a, 2b of the cavities 17a, 17b of the container and cover members 9, 11 abut one another at an interface 2c to form an enclosed internal space 8 (see FIGURE 7), to an open position shown in FIGURE 3, in which the cavity 17a of the container member 9 is accessible. As will be understood from FIGURES 6 and 7, the lip surfaces 2a, 2b are each provided with detent elements 4, 6 which engage with one another in the closed position to provide a releasable snap-fit fastening of the container and cover members 9, 11 in the closed position.
(2) Rotary or pivotable movement of the cover member 11 about the aperture 31 (pivot axis A-A) from a first angular position corresponding to the open position shown in FIGURE 3 to a second angular position shown in FIGURE 4A in which the cover member 11 is located behind the container member 9. The cover member 11 is not able to be pivoted about the pivot axis A-A from its closed position due to the interengagement of the lip surfaces 2a, 2b.

As shown in FIGURE 4B, when the cover member 11 is in the second angular position it is able to nest with the container member 9 by forward movement in the direction of arrow B. In other words, the concave cavity 17b of the shell-like cover member 11 is able to slidingly receive the convex rear surface of the container member 9. In the nesting position, an interference fit is formed between the container and cover members 9, 11 to releasable fasten them in the nesting position.

When the container and cover members 9,11 adopt the nesting configuration, an ergonomic unit is formed which is able to be easily, and comfortably, held by a hand 90 of a patient 92, as shown in FIGURE 1. In this mode, the outer casing part 3 is able to act as a holder or applicator of the drug delivery device 1.

As shown in FIGURE 5, for example, the outer surface 33 at the rear side of the container member 9 is provided with a window 35. As will be understood from FIGURES 4A and 4B, the window 35 is covered by the cover member 11 when in its second angular position. If the cover member 11 is transparent or semi-transparent, the window 35 is then visible through the cover member 11 when in its second angular position.

If need be, an indexing or detent mechanism could be provided for indexing the cover member 11 in one or more predetermined angular positions about the pivot axis A-A, for instance the first and/or second angular positions and/or angular positions therebetween. The user would then have a tactile feedback indicating that the cover member 11 is in the correct angular position, e.g. for nesting with the container member 9. One way of achieving the indexing mechanism would be a male-and-female arrangement in which male (or female) surface features are formed in the outer surface 33 of the container member 9 at the required angular dispositions about the pivot axis A-A and one or more complementary female (or male) surface features are correspondingly arranged on the inner surface of the hinge 13 about the knob 15. When the male feature is located in the, or one of the, female features (or vice-versa), the cover member 11 is indexed in a predetermined angular position. To move the cover member 11 to a new angular position, rotation of the cover member 11 about the pivot axis A-A causes disconnection of the first indexing connection until the male and female features re-engage at a new angular position of the cover member 11

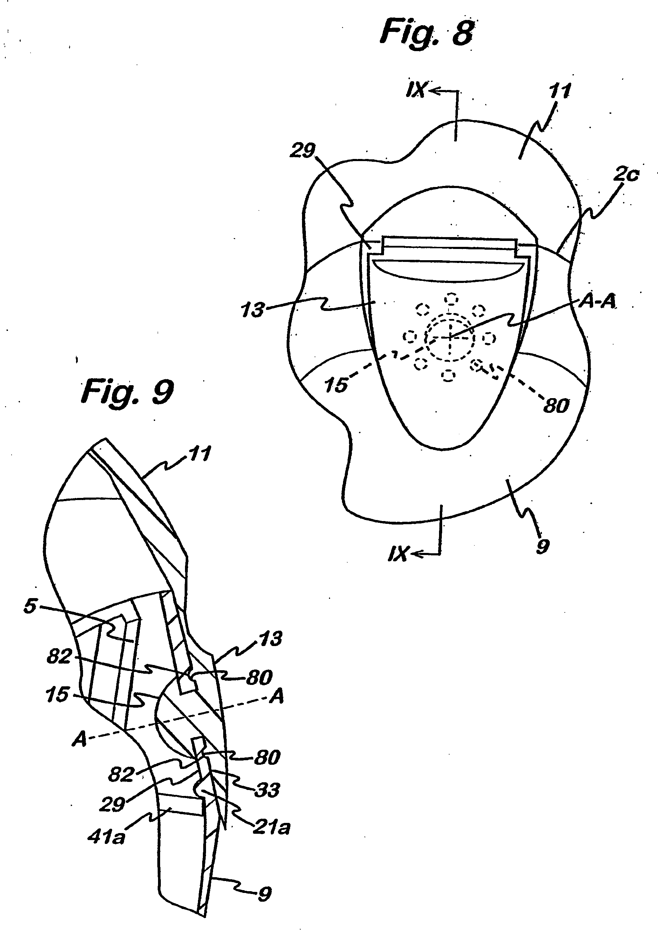

By way of example, a "ball-and-socket" indexing mechanism is shown in FIGURES 8 and 9 in which a circular array of protrusions 80 is formed on the inner surface of the hinge 13 co-axially with the knob 15, and a complementary circular array of sockets 82 is formed in the outer surface 33 of the bevel 29 on the container member 9 co-axially with the pivot axis A-A. When the protrusions 80 are located in the complementary sockets 82, the cover member 11 is indexed in one of a plurality of different predetermined angular positions it can adopt about the pivot axis A-A, for instance the first angular position of FIGURES 2 and 3 or the second angular position of FIGURES 4A and 4B. The cover member 11 is then able to be indexed in a new predetermined angular position by pivoting it about the pivot axis A-A to disconnect and re-engage the protrusions 80 and sockets 82, e.g. from the first angular position to the second angular position and vice-versa.

In an alternative embodiment, an indexing mechanism may be provided which indexes the cover member 11 in an end angular position between the first and second angular positions mentioned previously such that the cover member 11 does not cover the window 35 in the container member 9, thereby allowing the window 35 to be viewed by a user of the intranasal device 1.

### Canister Unit

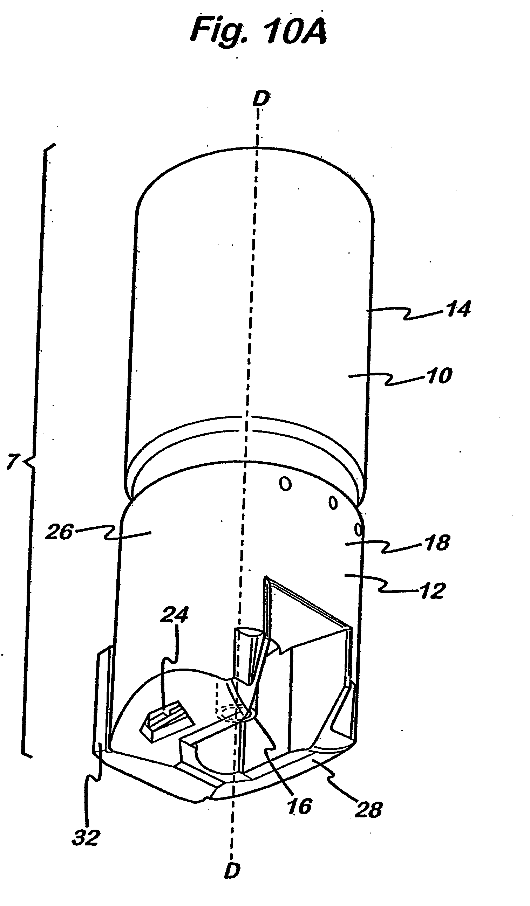

Turning attention now to FIGURES 10A to 10D, the canister unit 7 comprises an aerosol canister 10 of standard type which contains a drug formulated in a fluid propellant, e.g. a liquefied gas propellant such as a hydrofluoro alkane (HFA), for instance 1,1,1,2-tetrafluoroethane (CF₃CH₂F) (known as "HFA 134a") or 1,1,1,2,3,3,3-heptafluoro-n-propane (CF₃CHFCF₃) (known as "HFA 227") or a mixture thereof. The drug is typically for the treatment or prophylaxis of respiratory diseases or disorders, for example rhinitis. The drug may also be for the treatment or prophylaxis of other types of disease or disorder through systemic action of the drug. The canister unit 7 further comprises a dose counter head 12.

The canister 10 has a metal canister body 14 which, as known in the art, e.g. from metered dose inhalers (MDIs), has an open end which is capped by a valve assembly including a valve stem 16 which is mounted so it is movable relative to the canister body 14, between a retracted or open position relative to the canister body 14 in which the drug formulation is discharged from the canister 10 through the valve stem 16, and an extended or closed position relative to the canister body 14 in which the drug formulation is prevented from being discharged from the canister 10.

As known in the art, the valve assembly includes a biasing mechanism such as a spring (not shown) for biasing the valve stem 16 to the closed position. The valve assembly may be such as to further include a metering mechanism which operates so that a metered dose of the drug formulation is discharged when the valve stem 16 is in its open position. Typically, the valve assembly will have a metering chamber of fixed volume which in the closed position of the valve stem 16 is sealed from the external environment but in fluid communication with the canister body 14 whereby the metering chamber is filled with the drug formulation, and which in the open position of the valve stem is sealed from the canister body 14 and its contents, but placed in fluid communication with the external environment so that the metered dose of the drug formulation in the chamber is discharged to the external environment through the valve stem 16.

In this embodiment of the invention the valve assembly is a metering valve which dispenses a metered dose of the drug formulation per actuation thereof. A suitable metering valve is disclosed in WO98/29321.

The dose counter head 12 of the canister unit 7 has a hollow plastics body 18 of a plastics material which is fixedly secured to the canister 10 over the outlet end of the canister 10 having the valve assembly. The dose counter body 18 is fixed to the canister 10 to prevent it being taken off the canister 10, although it is free to rotate about a longitudinal axis D-D of the canister unit 7. The dose counter body 18 may be fixed to the canister 10 in the manner described and shown in WO01/28887 (Glaxo/Brand *et al*).

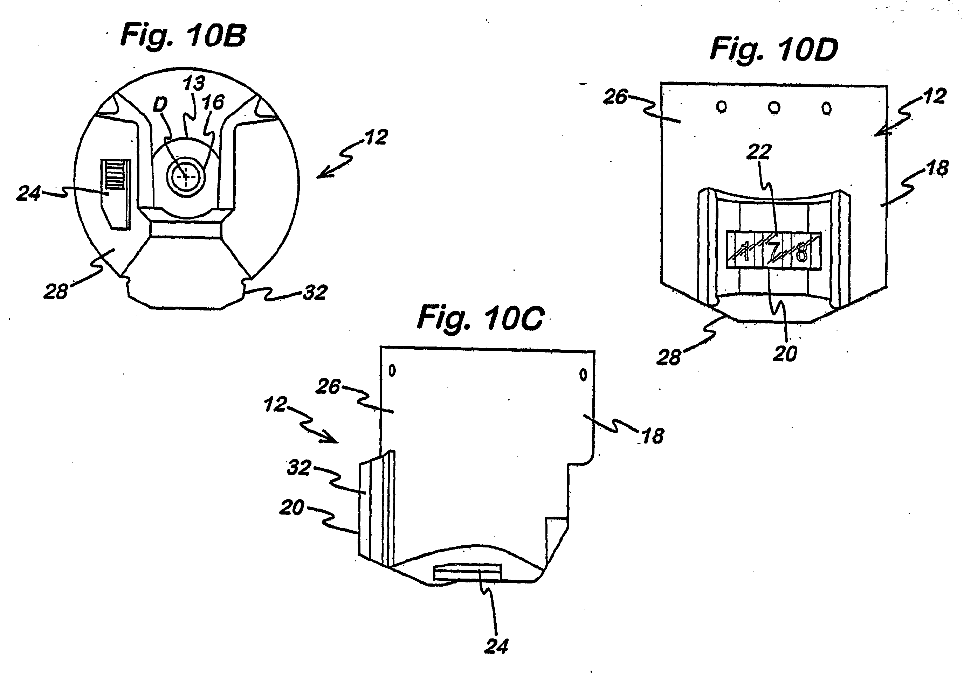

As best shown in FIGURE 10D, the dose counter body 18 is formed with a display window 20. The dose counter head 12 further includes a dose counting mechanism (not shown) in the body 18 which, when actuated, advances a counter 22 thereof located in the window 20. When the counter 22 is advanced it results in the dose count shown thereby in the window 20 either being incremented to indicate the number of doses dispensed or, more preferably, decremented to show the number of doses left in the canister 10. The dose counter mechanism can take one of the forms described and shown in WO98/56444 (Glaxo/Rand *et al*) or Applicant's co-pending International patent application No. PCT/EP03/06466 (Applicant's Ref: PB60210).

An aperture 24 is provided in the outer surface of the dose counter body 18 to enable a driver to engage with the dose counting mechanism to advance the counter 22 when a dose of the drug formulation is dispensed from the canister 10 by the inner actuating part 5, as will be described in more detail hereinafter.

As shown in FIGURES 10A and 10B, the dose counter body 18 comprises a skirt-like lower section 26 and a U-shaped upper section 28. The dose counter body 18 also has a central opening 13 to the U-shaped upper section 28 through which the valve stem 16 protrudes. It will further be seen from FIGURES 10C and 10D that the dose counter window 20 is formed in a protrusion 32 in the outer peripheral surface of the U-shaped upper section 28.

### Inner Actuating Part

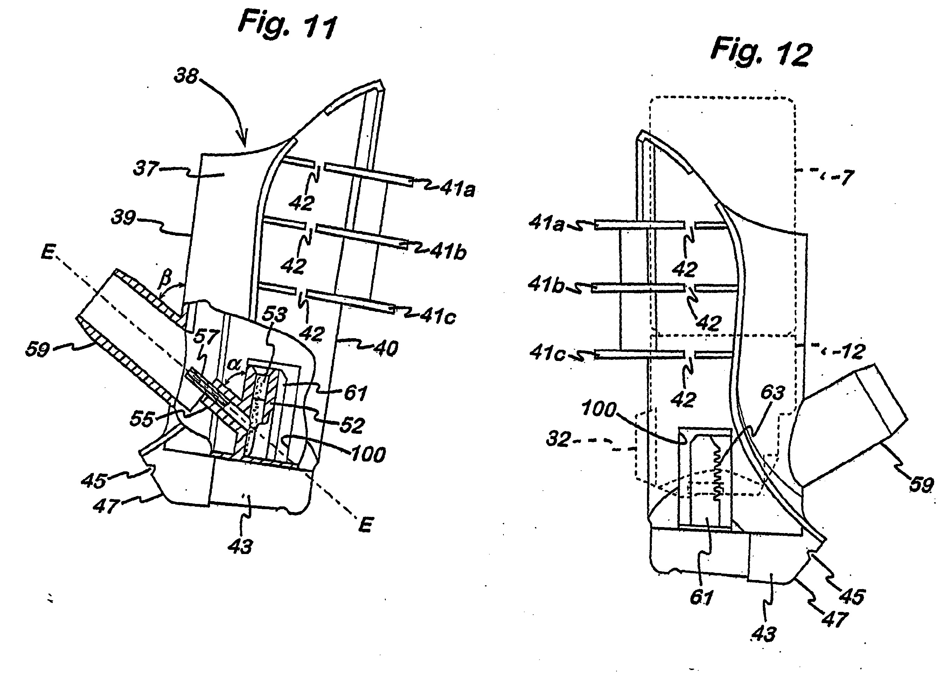

Attention is now turned to FIGURES 11 to 15B which show the inner actuating part 5. The inner actuating part 5 is of a plastics material, preferably polypropylene, made by a moulding process, preferably by injection moulding.

As will be seen, the inner actuating part 5 is of tubular construction having a main body 37 defining an axially-oriented cavity 38. The main body 37 has an outer surface 39 having a rear section 40 of shape and size which is complementary to the shape and size of the inner surface 19 of the container member cavity 17a, thereby enabling the inner actuating part 5 to fit snugly in the container member cavity 17a, as shown in FIGURES 3 and 7, for example. More particularly, the rear section 40 of the main body outer surface 39 is provided with a series of axially-spaced, circumferential ribs 41 a-c which act as spacers to position the inner actuating part 5 along a vertical axis C-C in the container member 9, as shown in FIGURES 6 and 7. Moreover, as further shown in FIGURE 7, the uppermost circumferential rib 41a is adapted to be snap fit underneath the locking rib 21 a on the inner surface 19 of the container member 9.

FIGURES 11 to 13 show that the circumferential ribs 41a-c are intersected by longitudinal slots 42, 44. The longitudinal slots 42; 44 are positioned and sized so as to co-operate with the longitudinal ribs 27a, 27b on the inner surface 19 of the container member 9 of the outer casing part 3 to prevent rotation of the inner actuating part 5 in the container member 9 of the outer casing part 3.

It will further be seen that the inner actuating part 5 has a foot structure 43 which, as shown in FIGURE 7, stands on a base surface 60 of the cavity 17a of the container member 9. The foot structure 43 includes a notch 45 on its forwardmost surface 47 which engages with the locking rib 21b at the lower end 25 of the cavity 17a of the container member 9. When the locking features 21a, 21b; 41 a, 45 of the container member 9 and the inner actuating part 5 respectively engage with one another, the inner actuating part 5 is releasably fixed in place in the container member 9. Only when a sufficient separation force is applied is the inner actuating part 5 released from the container member 9.

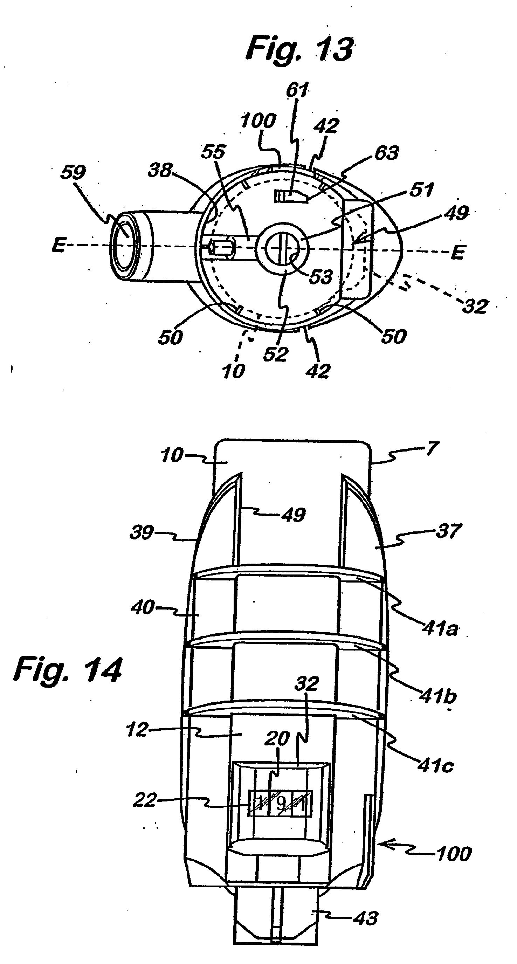

The rear section 40 of the main body 37 of the inner actuating part 5 has a longitudinally extending guide slot 49. The guide slot 49 is sized to slidingly receive the protrusion 32 on the dose counter body 18. The canister unit 7 can only be inserted into the inner actuating part 5 when the protrusion 32 is aligned with the guide slot 49 in the inner actuating part 5, as shown in FIGURE 6. Thus, the guide slot 49 acts as a track along which the protrusion 32 slidingly moves to insert the canister unit 7 into the inner actuating part 5 and retract it therefrom. The guide slot 49 also co-operates with the protrusion 32 to act as an anti-rotation feature which prevents rotation of the dose counter head 12 in the inner actuating part 5, as will be understood by reference to FIGURES 12 and 13.

As shown in FIGURE 13, the inner actuating part 5 also has longitudinal spacers 50 arranged circumferentially about the inner surface of the cavity 38 so that the canister unit 7 is generally co-axially mounted in the inner actuating part 5.

At the base of the cavity 38 of the inner actuating part 5 there is provided a hollow support 51 (so-called "stem block") having a sleeve 52 with a bore 53 sized to receive the valve stem 16 of the canister 10. It will be appreciated that when the canister unit 7 is inserted axially into the cavity 38, the U-shaped upper section 28 of the dose counter head 12 encloses the support 51 on three sides thereof. The hollow support 51 includes a hollow extension 55 which extends outwardly at an acute angle α to the outer periphery of the sleeve 52. The extension 55 has a bore 57 which opens into the bore 53 of the sleeve 52.

The bore 57 of the extension 55 is co-axial with a longitudinal axis E-E of a nozzle 59 which extends forwardly and upwardly at an acute angle β to the outer peripheral surface of the body 37 of the inner actuating part 5. The acute angles α, β may be the same, or substantially the same. As shown in FIGURE 1, the nozzle 59 is shaped and sized to be received in a nostril 94 of a nose 96 of the patient 92.

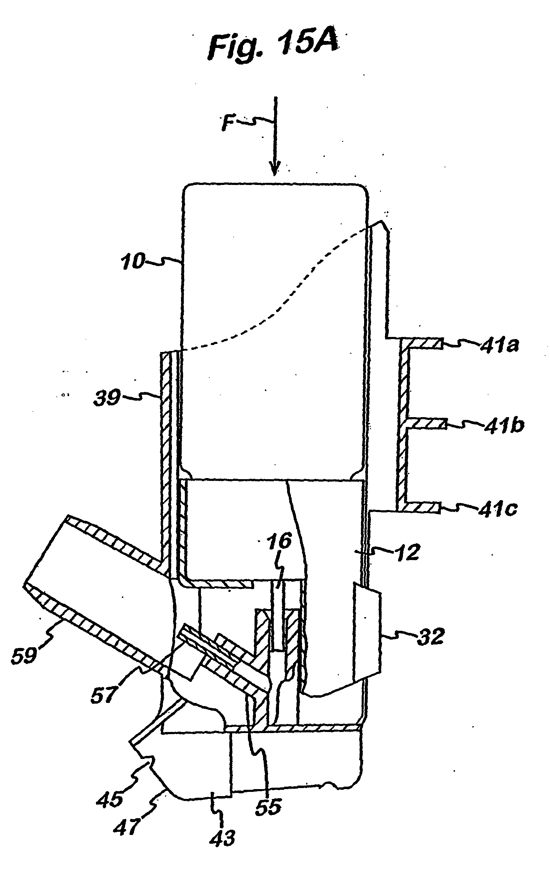

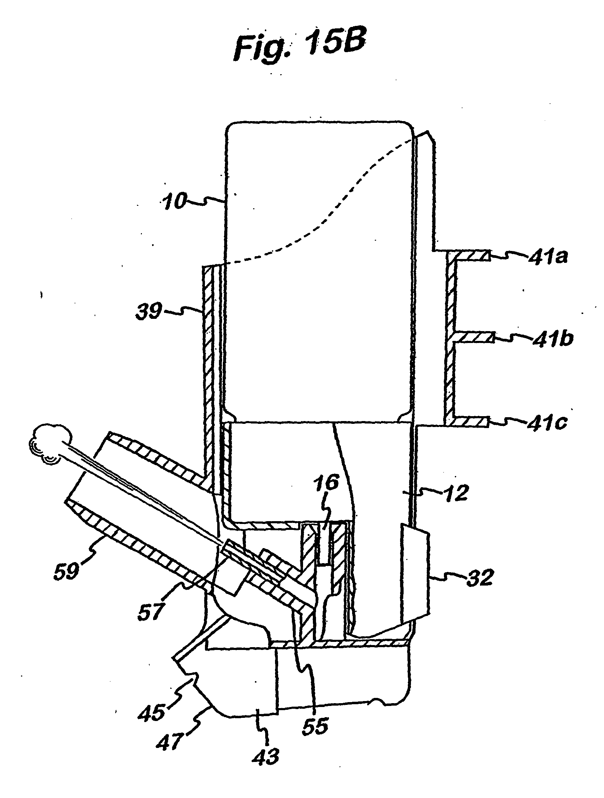

FIGURE 15A shows the canister unit 7 mounted in the inner actuating part 5 with the valve stem 16 held in the bore 53 of the sleeve 52 of the hollow support 51. As will be understood by comparing FIGURES 15A and 15B, in operation the canister body 14 (and counter head 12) is depressed in the direction of arrow F relative to the inner actuating part 5 and the valve stem 16, the stem 16 being held stationary in the hollow support 51. This relative movement of the canister body 14 to the valve stem 16 causes a metered dose of the drug formulation to be discharged through the valve stem 16 into the bore 53 of the sleeve 52. The drug formulation is then channeled by the extension 55 into the nozzle 59 for delivery to the patient's nostril 94. The biasing mechanism in the valve assembly causes the canister body 14 to re-adopt the inoperative position shown in FIGURE 15A on release of the depressing force F ready for the next actuation cycle.

It can therefore be seen from the above that the inner actuating part 5 contains all of the actuating elements for causing actuation of the canister 10 so that the drug formulation is discharged therefrom into the patient 92. In other words, the inner actuating part 5 is adapted to actuate the canister 10 when separate from the outer casing part 3.

Moreover, the base of the cavity 38 of the inner actuating part 5 carries a rack-like post 61 having a serrated outer profile 63 (teeth) which co-operates with the dose counter mechanism such that on depression of the canister body 14 in the inner actuating part 5 to its operative position shown in FIGURE 15B, the rack-like post 61 drives a cog system in the dose counter mechanism (see WO98/56444 and PCT/EP03/06466 *supra*) so that the counter 22 in the display 20 is advanced to reflect that the canister 10 has been actuated to dispense a dose of the drug formulation.

It will be observed from FIGURES 11 to 13, for example, that the main body 37 of the inner actuating part 5 has an aperture 100 through a side thereof which is aligned with the rack-like post 61. The aperture 100 is left by that member of the inner actuating part mould assembly used to form the post 61. In this regard, it will be gathered from FIGURE 13 that the post 61 does not register with the nozzle 59. That is to say, the post 61 is offset to the nozzle axis E-E such that it would not be possible to have the mould member for the post 61 extracted through the nozzle 59. This is because the nozzle diameter has to be small enough to be insertable into the patient's nostril 94 and the post 61 is positioned to one side of the hollow support 51 which is in registration with the nozzle 59 for fluid communication therebetween.

As will be realised, the rack-like post 61 can be replaced by any other dose counter mechanism driver structure as dictated by the form of the dose counter mechanism. Accordingly, the dose counter mechanism driver can take the form of another type of mechanical element or a non-mechanical element, for example. Again, reference may be had to WO98/56444 *supra.*

It can be seen from FIGURE 2 that once the canister unit 7 is assembled with the inner actuating part 5 and this assembly snap-fitted into the outer casing part 3, the outer casing part 3 acts as a protective casing for the canister unit 7 when in its closed state as it prevents actuation of the canister unit 7 and shields the nozzle 59 of the inner actuating part 5.

### Operation

To use the intranasal device 1, the patient 92 moves the cover member 11 of the outer casing part 3 from the closed position shown in FIGURE 2 to the open position shown in FIGURE 3. The cover member 11 is then pivoted to the second angular position shown in FIGURE 4A and then nested with the container member 9, as shown in FIGURE 4B. Then, as shown in FIGURE 1, the patient 92 grips the outer casing member 3 in one hand 90 and inserts the nozzle 59 into the nostril 94. The patient 92 then actuates the device 1 by depressing the canister body 14 into the inner actuating part 5 relative to the valve stem 16 with the index finger 98. This results in a metered dose of the drug formulation being delivered to the nostril.

Actuation of the device 1 can be confirmed by the patient 92 observing whether the dose counter 22 has been advanced (decremented/incremented). The patient 92 then closes the outer casing part 3 to protect the inner actuating part 5 and canister unit 7 until the next dose is required to be dispensed.

As will be seen from FIGURE 1, when the outer casing part 3 is in its open state it acts as a holder/applicator for the patient 92 in the sense that the patient 92 is able to grip the outer casing part 3 in one hand 90 and to depress the canister body 14 (and counter head 12) into the inner actuating part 5 with the index finger 98 to cause a dose of the drug formulation to be dispensed through the nozzle 59 and the counter 20 to be incremented/decremented.

One of the numerous advantages of the intranasal device 1 is the use of separable outer casing and inner actuating parts 3, 5. Recalling that the inner actuating part 5 contains all of the functional features for actuating the canister unit 7, both from a drug delivery and dose counting point of view, this is the only part which needs to be tested and submitted for regulatory approval. The outer casing part 3 does not affect the performance of the inner actuating part 5 in any way. This would be in contrast to the case where the outer and inner parts 3, 5 are integrally formed. In this instance, any change to the external shape and configuration after approval would necessitate a new application for approval since the alteration may have an adverse effect on the internal functional features, especially if the component is moulded from a plastics material.

So, the inner actuating part 5 is able to be designed first and then the decorative outer protective casing part 3 designed afterwards. In this way, the inner actuating part 5 can be tested and submitted for regulatory approval before the outer casing part 3 is finalised. This shortens the lead time for developing an approved drug delivery device. Moreover, the outer casing part 3 can be redesigned to maintain a contemporary appearance etc. without requiring a new round of regulatory tests.

Appropriate drugs (or medicaments) for use in the present invention, for instance forming part of a pharmaceutical aerosol formulation having a fluid propellant, e.g. a HFA propellant, such as HFA 134a or HFA 227, may be selected from, for example, analgesics, e.g., codeine, dihydromorphine, ergotamine, fentanyl or morphine; anginal preparations, e.g., diltiazem; antiallergics, e.g., cromoglycate (e.g. as the sodium salt), ketotifen or nedocromil (e.g. as the sodium salt); antiinfectives e.g., cephalosporins, penicillins, streptomycin, sulphonamides, tetracyclines and pentamidine; antihistamines, e.g., methapyrilene; anti- inflammatories, e.g., beclomethasone (e.g. as the dipropionate ester), fluticasone (e.g. as the propionate ester), flunisolide, budesonide, rofleponide, mometasone (e.g. as the furoate ester), ciclesonide, triamcinolone (e.g. as the acetonide), 6α, 9α-difluoro-11β-hydroxy-16α-methyl-3-oxo-17α-propionyloxy-androsta-1,4-diene-17β-carbothioic acid S-(2-oxo-tetrahydro-furan-3-yl) ester or 6α, 9α-Difluoro-17α-[(2-furanylcarbonyl)oxy]-11β-hydroxy-16α-methyl-3-oxo-androsta-1,4-diene-17β-carbothioic acid S-fluoromethyl ester; antitussives, e.g., noscapine; bronchodilators, e.g., albuterol (e.g. as free base or sulphate), salmeterol (e.g. as xinafoate), ephedrine, adrenaline, fenoterol (e.g. as hydrobromide), formoterol (e.g. as fumarate), isoprenaline, metaproterenol, phenylephrine, phenylpropanolamine, pirbuterol (e.g. as acetate), reproterol (e.g. as hydrochloride), rimiterol, terbutaline (e.g. as sulphate), isoetharine, tulobuterol or 4-hydroxy-7-[2-[[2-[[3-(2-phenylethoxy)propyl]sulfonyl]ethyl]amino]ethyl-2(3H)-benzothiazolone; PDE4 inhibitors e.g. cilomilast or roflumilast; leukotriene antagonists e.g. montelukast, pranlukast and zafirlukast; [adenosine 2a agonists, e.g. 2R,3R,4S,5R)-2-[6-Amino-2-(1S-hydroxymethyl-2-phenyl-ethylamino)-purin-9-yl]-5-(2-ethyl-2H-tetrazol-5-yl)-tetrahydro-furan-3,4-diol (e.g. as maleate)]; [α4 integrin inhibitors e.g. (2S)-3-[4-({[4-(aminocarbonyl)-1-piperidinyl]carbonyl}oxy)phenyl]-2-[((2S)-4-methyl-2-{[2-(2-methylphenoxy) acetyl]amino}pentanoyl)amino] propanoic acid (e.g. as free acid or potassium salt)], diuretics, e.g., amiloride; anticholinergics, e.g., ipratropium (e.g. as bromide), tiotropium, atropine or oxitropium; hormones, e.g., cortisone, hydrocortisone or prednisolone; xanthines, e.g., aminophylline, choline theophyllinate, lysine theophyllinate or theophylline; therapeutic proteins and peptides, e.g., insulin or glucagons. It will be clear to a person skilled in the art that, where appropriate, the medicaments may be used in the form of salts, (e.g., as alkali metal or amine salts or as acid addition salts) or as esters (e.g., lower alkyl esters) or as solvates (e.g., hydrates) to optimise the activity and/or stability of the medicament and/or to minimise the solubility of the medicament in the propellant.

Preferably, the medicament is for the treatment of inflammatory and/or allergic conditions of the nasal passages such as rhinitis, e.g. seasonal and perennial rhinitis as well as other local inflammatory conditions such as asthma, COPD and dermatitis. Preferably, the medicament is an anti-inflammatory compound for the treatment of asthma or rhinitis.

It is to be understood that the exemplary embodiments of the present invention outlined above are for the purposes of illustration only, and that the invention can be modified, varied and take on other guises within the scope of the appended claims. Mindful of this, the use of reference numerals in the claims is not to be taken as having a limiting effect on the scope of the claims.

For the avoidance of doubt, the use herein of terms such as "generally", "substantially", "about" and the like when referring to a parameter of the invention is meant to include the absolute parameter.

## Claims

1. A container (3) having a first part (11), a second part (9) and a hinge (13) through which the first and second parts are hingeably connected so that the parts are hingeable relative to one another between a first position which places the container in a closed state and a second position which places the container in an open state, **characterised in that** the first and second parts are further pivotally connected (15, 31) so that the parts are able to be pivoted relative to one another to different angular positions.

2. The container of claim 1 adapted such that the first and second parts are not pivotal relative to one another when the container is in the closed state.

3. The container of claim 2 wherein in the closed state the first and second parts interengage one another so as to prevent pivotal movement therebetween.

4. The container of claim 3 wherein the interengagement is between respective lip structures (2a, 2b) of the first and second parts.

5. The container of any one of the preceding claims wherein the hinge is a living hinge.

6. The container of any one of the preceding claims wherein the first and second parts have locking elements (4,6) which interengage in the closed state of the container to lock the container in the closed state.

7. The container of claim 6 wherein the locking elements form a snap fit connection of the first and second parts in the closed state of the container.

8. The container of any one of the preceding claims wherein the hinge is pivotally mounted (15, 31) to the second part for enabling the relative pivotal movement of the first and second parts.

9. The container of any one of the preceding claims wherein the hinge is statically mounted to the first part.

10. The container of claims 8 and 9 wherein the hinge forms an integral part of the first part and is pivotally mounted to the second part.

11. The container of any one of the preceding claims wherein the first part is pivotal to a first angular position disposed behind the second part.

12. The container of claim 11 wherein the first part is pivotal to the first angular position after the container is in the open state.

13. The container of claim 11 or 12 wherein in the first angular position the first and second parts are adapted to nest together in a nesting state.

14. The container of any one of claims 11 to 13 wherein the second part has a rear surface in which is provided a reading feature (35), the first part covers the reading feature in the first angular position and has a viewing feature to enable the reading feature to be viewed through the first part when in the first angular position.

15. The container of claim 14 wherein the first part is at least in part made from a material sufficiently transparent to enable the reading feature to be viewed therethrough when the first part is in the first angular position.

16. The container of any one of the preceding claims further having a detent mechanism (80, 82) for detenting the first and second parts in different angular positions.

17. The container of claims 11 and 16 wherein the second part has a rear surface in which is provided a reading feature (35) and the detent mechanism is adapted to detent the first part in the first angular position, which first angular position provides an unimpeded view of the reading feature on the second part.

18. The container of any one of claims 14, 15 or 17 wherein the reading feature is a window for viewing the contents of the container.

19. The container of any one of the preceding claims wherein the first and second parts form an enclosing structure when in the closed state.

20. The container of claim 19 wherein the enclosing structure is an outer structure (3) and wherein the container includes an inner part (5) releasably, fixably securable in one of the first and second parts so as to be enclosed by the outer structure.

21. The container of claim 20 wherein the inner part is adapted to be snap-fitted in one of the first and second parts.

22. The container of claim 20 or 21 in which the inner part is securable in the second part.

23. The container of claim 20, 21 or 22 in which the inner part is adapted to hold a product (7) in the container.

24. The container of claim 23 in which the product is a package with contents to be dispensed.

25. The container of claim 24 in which the inner part is a dispenser with a dispensing mechanism (51) for dispensing the contents of the package.

26. The container of claim 25 in which the dispensing mechanism operates through relative movement between the dispenser and the package when the dispenser is fixedly secured in the outer structure.

27. The container of claim 25 or 26 in which the dispenser has a delivery nozzle (59) through which the contents of the package are dischargeable.

28. The container of any one of claims 25 to 27 in which the dispenser is an intranasal dispenser (1).

29. The container of any one of claims 25 to 28 when appendant on claim 14, 15 or 17 including the package, the package and dispenser forming a dispenser assembly, the package containing multiple doses of the contents and the dispenser assembly having a dose counter (12) for counting the number of doses dispensed from the package, the dose counter being aligned with the reading feature when the dispenser assembly is secured in the container.

30. The container of any one of claims 25 to 28 wherein the package is an aerosol canister with fluidic contents and having a valve (16) openable by the dispensing mechanism on relative movement of the canister to the dispenser for release of a dose of the fluidic contents from the dispenser.

31. The container of claim 30 in which the valve has a stem (16) through which the fluidic contents is dispensed from the dispenser on relative movement thereof to the canister and in which the dispenser has a stand (51) adapted to receive the stem in static relation thereto whereby the canister is able to be moved relative to the stem when received in the stand for dispensing of the fluidic contents.

32. The container of any one of claims 24 to 28, 30 and 31 including the package.

33. The container of claim 32 in which the contents of the package is a pharmaceutical composition.

34. A medicament dispensing system having the container of any one of the preceding claims.

## Patentansprüche

1. Behälter (3) mit einem ersten Teil (11), einem zweiten Teil (9) und einem Gelenk (13), durch welches die ersten und zweiten Teile gelenkig verbunden sind, so dass die Teile relativ zueinander zwischen einer ersten Position, die den Behälter in einem geschlossenen Zustand anordnet, und einer zweiten Position, die den Behälter in einem offenen Zustand anordnet, gelenkig sind, **dadurch gekennzeichnet, dass** die ersten und zweiten Teile ferner schwenkbar verbunden (15, 31) sind, so dass die Teile relativ zueinander in unterschiedliche Winkelpositionen geschwenkt werden können.

2. Behälter nach Anspruch 1, der derart ausgebildet ist, dass die ersten und zweiten Teile nicht relativ zueinander schwenkbar sind, wenn der Behälter in dem geschlossenen Zustand ist.

3. Behälter nach Anspruch 2, bei dem die ersten und zweiten Teile in dem geschlossenen Zustand ineinander eingreifen, um eine Schwenkbewegung dazwischen zu verhindern.

4. Behälter nach Anspruch 3, bei dem der Eingriff ineinander zwischen jeweiligen Lippenstrukturen (2a, 2b) der ersten und zweiten Teile ist.

5. Behälter nach einem der vorhergehenden Ansprüche, bei dem das Gelenk ein Wohnscharnier ist.

6. Behälter nach einem der vorhergehenden Ansprüche, bei dem die ersten und zweiten Teile Verriegelungselemente (4, 6) aufweisen, die in dem geschlossenen Zustand mit dem Behälter in Eingriff gelangen, um den Behälter in dem geschlossenen Zustand zu verriegeln.

7. Behälter nach Anspruch 6, bei dem die Verriegelungselemente eine Schnappverschlussverbindung der ersten und zweiten Teilen in dem geschlossenen Zustand des Behälters ausbilden.

8. Behälter nach einem der vorhergehenden Ansprüche, bei dem das Gelenk an dem zweiten Teil schwenkbar angebracht (15, 31) ist, um die relative Schwenkbewegung der ersten und zweiten Teile zu ermöglichen.

9. Behälter nach einem der vorhergehenden Ansprüche, bei dem das Gelenk statisch an dem ersten Teil angebracht ist.

10. Behälter nach Anspruch 8 und 9, bei dem das Gelenk einen integralen Teil des ersten Teils ausbildet, und schwenkbar an dem zweiten Teil angebracht ist.

11. Behälter nach einem der vorhergehenden Ansprüche, bei dem das erste Teil in eine erste Winkelposition schwenkbar ist, die hinter dem zweiten Teil angeordnet ist.

12. Behälter nach Anspruch 11, bei dem das erste Teil in die erste Winkelposition schwenkbar ist, nachdem der Behälter in dem offenen Zustand ist.

13. Behälter nach Anspruch 11 oder 12, bei dem die ersten und zweiten Teile in der ersten Winkelposition geeignet sind, in einem Verschachtelungszustand zusammen verschachtelt zu sein.

14. Behälter nach einem der Ansprüche 11 bis 13, bei dem das zweite Teil eine hintere Oberfläche aufweist, in der ein Lesemerkmal (35) vorgesehen ist, wobei das erste Teil das Lesemerkmal in der ersten Winkelposition bedeckt und ein Sichtmerkmal aufweist, um zu ermöglichen, dass das Lesemerkmal durch das erste Teil angesehen wird, wenn es in der ersten Winkelposition ist.

15. Behälter nach Anspruch 14, bei dem das erste Teil zumindest teilweise aus einem ausreichend transparenten Material hergestellt ist, um zu ermöglichen, dass das Lesemerkmal **dadurch** angesehen wird, wenn das erste Teil in der ersten Winkelposition ist.

16. Behälter nach einem der vorhergehenden Ansprüche, ferner mit einem Rastmechanismus (80, 82), zum Einrasten der ersten und zweiten Teile in unterschiedlichen Winkelposition.

17. Behälter nach einem Ansprüche 11 und 16, bei dem das zweite Teil eine hintere Oberfläche aufweist, in der ein Lesemerkmal (35) vorgesehen ist, und wobei der Rastmechanismus geeignet ist das erste Teil in der ersten Winkelposition einzurasten, wobei diese erste Winkelposition einen ungehinderten Blick auf das Lesemerkmal an dem zweiten Teil vorsieht.

18. Behälter nach einem der Ansprüche 14, 15 oder 17, bei dem das Lesemerkmal ein Fenster zum Ansehen der Inhalte des Behälters ist.

19. Behälter nach einem der vorhergehenden Ansprüche, bei dem die ersten und zweiten Teile eine umschließende Struktur ausbilden, wenn sie in dem geschlossenen Zustand sind.

20. Behälter nach Anspruch 19, bei dem die umschließende Struktur eine äußere Struktur (3) ist, und wobei der Behälter ein inneres Teil (5) umfasst, das lösbar, fest in einem der ersten und zweiten Teile befestigbar ist, um durch die äußere Struktur umschlossen zu sein.

21. Behälter nach Anspruch 20, bei dem das innere Teil geeignet ist, durch Einschnappen in eines der ersten und zweiten Teile eingepasst zu werden.

22. Behälter nach Anspruch 20 oder 21, bei dem das innere Teil in dem zweiten Teil befestigbar ist.

23. Behälter nach Anspruch 20, 21 oder 22, bei dem das innere Teil geeignet ist, ein Produkt (7) in dem Behälter zu halten.

24. Behälter nach Anspruch 23, bei dem das Produkt eine Packung mit abzugebenden Inhalten ist.

25. Behälter nach Anspruch 24, bei dem das innere Teil ein Spendermechanismus (51) zum Spenden der Inhalte der Packung ist.

26. Behälter nach Anspruch 25, bei dem der Spendermechanismus durch eine relative Bewegung zwischen dem Spender und der Packung betrieben wird, wenn der Spender in der äußeren Struktur fest befestigt ist.

27. Behälter nach Anspruch 25 oder 26, bei dem der Spender eine Abgabedüse (59) aufweist, durch die die Inhalte der Packung abgebbar sind.

28. Behälter nach einem der Ansprüche 25 bis 27, bei dem der Spender ein intranasaler Spender (1) ist.

29. Behälter nach einem der Ansprüche 25 bis 28, wenn sie von Anspruch 14, 15 oder 17 abhängig sind, einschließlich der Packung, wobei die Packung und der Spender eine Spenderanordnung ausbilden, die Packung multiple Dosen der Inhalte enthält und die Spenderanordnung einen Dosiszähler (12) zum Zählen der Anzahl von Dosen aufweist, die von der Packung abgegeben werden, wobei der Dosiszähler mit dem Lesemerkmal ausgerichtet ist, wenn die Spenderanordnung in dem Behälter befestigt ist.

30. Behälter nach einem der Ansprüche 25 bis 28, bei dem die Packung ein Aerosolkanister mit fluiden Inhalten ist und ein Ventil (16) aufweist, das durch den Abgabemechanismus auf eine relative Bewegung des Kanisters zu dem Spender hin zu öffnen ist, zur Freigabe von einer Dosis der fluiden Inhalte aus dem Spender.

31. Behälter nach Anspruch 30, bei dem das Ventil einen Schaft (16) aufweist, durch den die fluiden Inhalte abgegeben werden, und wobei der Spender eine Stütze (51) aufweist, die geeignet ist den Schaft in einer statischen Beziehung dazu aufzunehmen, wodurch der Kanister relativ zu dem Schaft bewegt werden kann, wenn er in der Stütze aufgenommen ist, zur Abgabe der fluiden Inhalte.

32. Behälter nach einem der Ansprüche 24 bis 28, 30 und 31, einschließlich der Packung.

33. Behälter nach Anspruch 32, bei dem die Inhalte der Packung eine pharmazeutische Zusammensetzung ist.

34. Medikamentabgabesystem mit dem Behälter nach einem der vorhergehenden Ansprüche.

## Revendications

1. Récipient (3) comportant une première partie (11), une seconde partie (9) et une charnière (13) à travers laquelle la première et la seconde parties sont reliées de manière articulée de sorte que les parties peuvent s'articuler l'une par rapport à l'autre entre une première position qui place le récipient en condition fermée et une seconde position qui place le récipient en condition ouverte, **caractérisé en ce que** la première et la seconde parties sont en outre reliées de manière pivotante (15, 31) de sorte que les parties peuvent pivoter l'une par rapport à l'autre jusqu'à des positions angulaires différentes.

2. Récipient selon la revendication 1, adapté de manière à ce que la première et la seconde parties ne peuvent pas pivoter l'une par rapport à l'autre lorsque le récipient est dans la condition fermée.

3. Récipient selon la revendication 2, dans lequel dans la condition fermée, la première et la seconde parties entrent mutuellement en prise de manière à éviter le mouvement de pivotement entre celles-ci.

4. Récipient selon la revendication 3, dans lequel la mise en prise mutuelle s'effectue entre des structures formant lèvre respectives (2a, 2b) de la première et de la seconde parties.

5. Récipient selon l'une quelconque des revendications précédentes, dans lequel la charnière est une charnière d'ouverture.

6. Récipient selon l'une quelconque des revendications précédentes, dans lequel la première et la seconde parties présentent des éléments de verrouillage (4, 6) qui entrent mutuellement en prise dans la condition fermée du récipient pour verrouiller le récipient dans la condition fermée.

7. Récipient selon la revendication 6, dans lequel les éléments de verrouillage forment un emboîtement par pression de la première et de la seconde parties dans la condition fermée du récipient.

8. Récipient selon l'une quelconque des revendications précédentes, dans lequel la charnière est montée de manière pivotante (15, 31) sur la seconde partie pour permettre le mouvement de pivotement relatif entre la première et la seconde parties.

9. Récipient selon l'une quelconque des revendications précédentes, dans lequel la charnière est montée de manière statique sur la première partie.

10. Récipient selon les revendications 8 et 9, dans lequel la charnière forme une seule pièce avec la première partie et est montée de manière pivotante sur la seconde partie.

11. Récipient selon l'une quelconque des revendications précédentes, dans lequel la première partie peut pivoter jusqu'à une première position angulaire disposée derrière la seconde partie.

12. Récipient selon la revendication 11, dans lequel la première partie peut pivoter jusqu'à la première position angulaire suite à la mise en condition ouverte du récipient.

13. Récipient selon la revendication 11 ou 12, dans lequel dans la première position angulaire, la première et la seconde parties sont adaptées pour s'emboîter mutuellement dans une condition emboîtée.

14. Récipient selon l'une quelconque des revendications 11 à 13, dans lequel la seconde partie présente une surface arrière dans laquelle est positionné un dispositif de lecture (35), la première partie recouvrant le dispositif de lecture dans la première position angulaire et comportant un dispositif de visualisation pour permettre la visualisation du dispositif de lecture à travers la première partie lorsqu'elle est dans la première position angulaire.

15. Récipient selon la revendication 14, dans lequel la première partie est au moins partiellement faite d'un matériau suffisamment transparent pour permettre de visualiser le dispositif de lecture à travers celle-ci lorsque la première partie est dans la première position angulaire.

16. Récipient selon l'une quelconque des revendications précédentes, comportant en outre un mécanisme d'encliquetage (80, 82) pour encliqueter la première et la seconde parties dans diverses positions angulaires.

17. Récipient selon les revendications 11 et 16, dans lequel la seconde partie présente une surface arrière dans laquelle est positionné un dispositif de lecture (35) et le mécanisme d'encliquetage est adapté pour encliqueter la première partie dans la première position angulaire, laquelle première position angulaire fournit une vue sans obstacle du dispositif de lecture positionné sur la seconde partie.

18. Récipient selon l'une quelconque des revendications 14, 15 ou 17, dans lequel le dispositif de lecture est une fenêtre permettant de visualiser le contenu du récipient.

19. Récipient selon l'une quelconque des revendications précédentes, dans lequel la première et la seconde parties forment une structure enveloppante lorsqu'elles sont dans la condition fermée.

20. Récipient selon la revendication 19, dans lequel la structure enveloppante est une structure externe (3) et dans lequel le récipient comprend une partie interne (5) pouvant être attachée de manière fixe et libérable dans l'une d'entre la première et la seconde parties, de manière à être enveloppée par la structure externe.

21. Récipient selon la revendication 20, dans lequel la partie interne est adaptée pour être emboîtée par pression dans l'une d'entre la première et la seconde parties.

22. Récipient selon la revendication 20 ou 21, dans lequel la partie interne peut être fixée dans la seconde partie.

23. Récipient selon la revendication 20, 21 ou 22, dans lequel la partie interne est adaptée pour retenir un produit (7) dans le récipient.

24. Récipient selon la revendication 23, dans lequel le produit est un emballage avec un contenu à distribuer.

25. Récipient selon la revendication 24, dans lequel la partie interne est un distributeur avec un mécanisme de distribution (51) destiné à distribuer le contenu de l'emballage.

26. Récipient selon la revendication 25, dans lequel le mécanisme de distribution fonctionne à travers un mouvement relatif entre le distributeur et l'emballage lorsque le distributeur est attaché de manière fixe dans la structure externe.

27. Récipient selon la revendication 25 ou 26, dans lequel le distributeur comporte un bec de distribution (59) à travers lequel peut être déchargé le contenu de l'emballage.

28. Récipient selon l'une quelconque des revendications 25 à 27, dans lequel le distributeur est un distributeur intranasal (1).

29. Récipient selon l'une quelconque des revendications 25 à 28 en ce qu'elles dépendent de la revendication 14, 15 ou 17, comprenant l'emballage, l'emballage et le distributeur formant un ensemble de distribution, l'emballage contenant plusieurs doses du contenu et l'ensemble de distribution comportant un compteur de doses (12) destiné à compter le nombre de doses distribuées par l'emballage, le compteur de doses étant aligné avec le dispositif de lecture lorsque l'ensemble de distribution est fixé dans le récipient.

30. Récipient selon l'une quelconque des revendications 25 à 28, dans lequel l'emballage est une cartouche d'aérosol avec un contenu fluide et comportant une soupape (16) ouvrable par le mécanisme de distribution suite au mouvement relatif de la cartouche par rapport au distributeur pour libérer une dose du contenu fluide depuis le distributeur.

31. Récipient selon la revendication 30, dans lequel la soupape comporte une tige (16) à travers laquelle le contenu fluide est distribué depuis le distributeur suite au mouvement relatif de celui-ci par rapport à la cartouche, et dans lequel le distributeur comporte un support (51) adapté pour recevoir la tige dans un rapport statique par rapport à celle-ci, moyennant quoi la cartouche peut être déplacée par rapport à la tige lorsqu'elle est reçue dans le support pour distribuer le contenu fluide.

32. Récipient selon l'une quelconque des revendications 24 à 28, 30 et 31, comprenant l'emballage.

33. Récipient selon la revendication 32, dans lequel le contenu de l'emballage est une composition pharmaceutique.

34. Système de distribution de médicament, comportant le récipient selon l'une quelconque des revendications précédentes.
